# FASCICULE DE BREVET EUROPEEN

(11) **EP 1 512 367 B1**
(45) Date de publication et mention de la délivrance du brevet: **07.11.2007**
(21) Numéro de dépôt: 04292131.2
(22) Date de dépôt: 03.09.2004
(51) Int. Cl.: A61B 1/303, A61B 1/32, A61B 17/42

(54) **Spéculum physiologique**
Physiologische Spekulum
Physiological speculum

(30) Priorité: 05.09.2003 FR 0310535
(43) Date de publication de la demande: 09.03.2005
(73) Titulaire: Letellier, Bernard, 29200 Brest (FR)
(72) Inventeur: Letellier, Bernard, 29200 Brest (FR)
(74) Mandataire: Faber, Jean-Paul

(56) Documents cités:
- WO-A-01/41627
- WO-A-95/31131
- US-A- 5 899 854
- US-A- 6 004 265
- US-A1- 2003 105 387

## Description

La présente invention se rapporte à un spéculum physiologique.

On connaît différents types de spéculum qui comprennent deux branches articulées l'une sur l'autre par une extrémité, chaque branche étant rectiligne et ayant une section arquée de manière à présenter une surface externe convexe et une surface interne concave.

Le document US 2003/0105387 décrit un spéculum comportant une première branche et une deuxième branche, chaque branche ayant une section arquée de manière que les faces intérieures en regard l'une de l'autre des deux branches soient concaves, tandis que les faces extérieures sont convexes.

Le spéculum connu sous l'appellation "CUSCO" présente deux pattes latérales dont l'une est solidaire d'une branche et l'autre de l'autre branche pour commander l'écartement desdites branches, des moyens étant prévus pour maintenir celles-ci plus ou moins écartées, lesdites branches étant généralement appelées valves.

Un autre spéculum connu sous le nom de "COLLIN" présente également deux branches rectilignes à section arquée afin de présenter une surface externe convexe et une surface interne concave, les branches étant articulées l'une sur l'autre par une extrémité et l'une des branches présentant, au voisinage de l'articulation, un filetage dans lequel se visse une vis dont une extrémité porte contre l'autre branche, tandis que l'autre extrémité est pourvue d'un molette afin de pouvoir commander l'écartement des branches et les maintenir dans différentes positions angulaires déterminées.

On a constaté que la voie génitale féminine n'est pas rectiligne mais présente une certaine courbe en raison d'un cravatage musculaire.

L'un des buts de l'invention est de réaliser un nouveau spéculum qui permet une mise en place plus facile et qui permet une meilleurs visualisation du col de l'utérus.

Le spéculum physiologique, selon l'invention, est du type comprenant deux branches, ou valves, articulées l'une sur l'autre par un extrémité, chaque branche ayant une section arquée de manière que les faces en regard l'une de l'autre soient concaves, tandis que les faces extérieures sont convexes, des moyens étant insérés entre les branches afin, d'une part, de commander leur écartement et, d'autre part, de les maintenir écartées dans la position angulaire désirée, ledit spéculum physiologique étant caractérisé en ce que les branches sont courbes.

Grâce à cette disposition, le spéculum est d'une mise en place plus aisée et s'adapte mieux à la cavité vaginale.

De plus, dans le cas où les cols sont très excentrés, en faisant pivoter le spéculum de 180° préalablement à sa mise en place, on peut avoir une visualisation plus aisée du col.

Un autre avantage de l'invention, dans le cas de spéculum réalisés en matière plastique et destinés à n'être utilisés qu'une seule fois, la courbure des branches donne une plus grande rigidité à celles-ci.

De préférence, les branches sont courbées de manière à présenter, à leur extrémité libre, un déport de l'ordre de 2 cm par rapport à l'autre extrémité.

Enfin, les branches peuvent être courbées du côté des moyens pour commander leur écartement ou au contraire du côté opposé.

L'invention va maintenant être décrite avec plus de détails en se référant à des modes de réalisation particuliers donnés à titre d'exemple seulement et représentés au dessins annexés.

Figure 1 est une vue en perspective d'un spéculum physiologique, selon l'invention.

Figure 2 est une vue en élévation du spéculum de la figure 1.

Figure 3 est une vue en élévation d'une variante de réalisation du spéculum des figures 1 et 2.

Figure 4 est une vue en élévation d'une variante de réalisation.

Figure 5 est une vue en élévation d'encore une variante de l'invention.

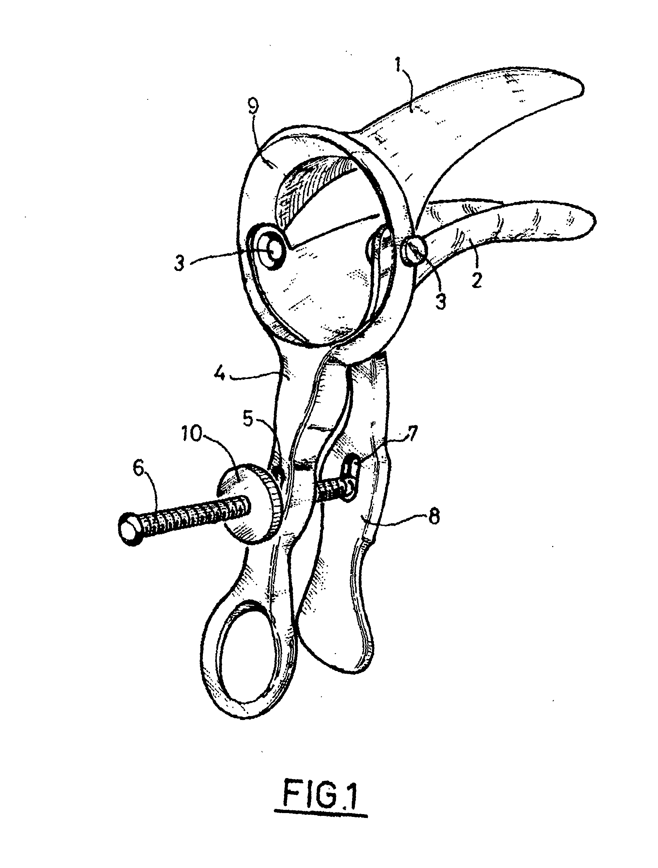

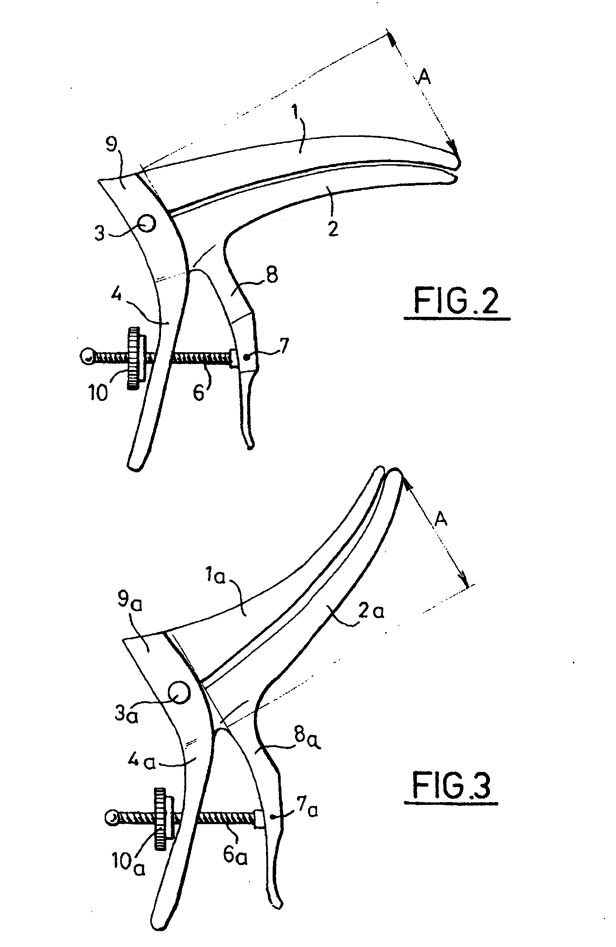

Aux figures 1 et 2, on a représenté un spéculum physiologique du type CUSCO comprenant deux branches 1 et 2 généralement appelées valves, la branche 1 étant terminée par un anneau 9 dans lequel s'articule sur des axes 3 l'extrémité correspondante de la branche 2.

L'anneau 9 est prolongé par une patte 4 légèrement inclinée vers l'arrière et percée d'une fenêtre 5 qui est traversée par une vis 6 qui s'articule sur un axe 7 porté par une seconde patte 8 solidaire de la seconde branche 2.

Sur la vis 6 est montée une molette 10 qui permet de caler les pattes dans des positions déterminées afin de maintenir les branches 1 et 2 écartées.

La branche 1 a une section arquée, la surface convexe étant tournée vers l'extérieur.

La branche 2 a également une section arquée, la surface convexe étant tournée vers l'extérieur, ainsi les faces tournées l'une en regard de l'autre sont concaves.

La longueur des branches 1 et 2 est de l'ordre de 12 à 15 centimètres et, comme on le voit plus nettement à la figure 2, les branches 1 et 2 sont courbes, le déport A de l'extrémité libre desdites branches 1 et 2 par rapport à l'extrémité adjacente à l'anneau 9, du côté opposé à la patte 4, est de l'ordre de 2 cm.

Le spéculum de la figure 2 s'utilise les pattes 4 et 8 tournées vers le bas.

A la figure 3, on a représenté une variante de l'invention et on a reporté sur cette figure les références utilisées aux figures précédentes pour désigner les parties correspondantes mais affectées de la lettre "a".

Dans ce mode de réalisation, le spéculum s'utilise les pattes 4a et 8a tournées vers le haut.

Les branches 1a et 2a sont ici courbées dans le sens inverse par rapport au mode de réalisation ; toutefois la courbure est sensiblement identique, pour une longueur des branches de l'ordre de 12 à 15 cm le déport A est de l'ordre de 2 cm par rapport à l'extrémité de la branche 2a adjacente à la patte 8a.

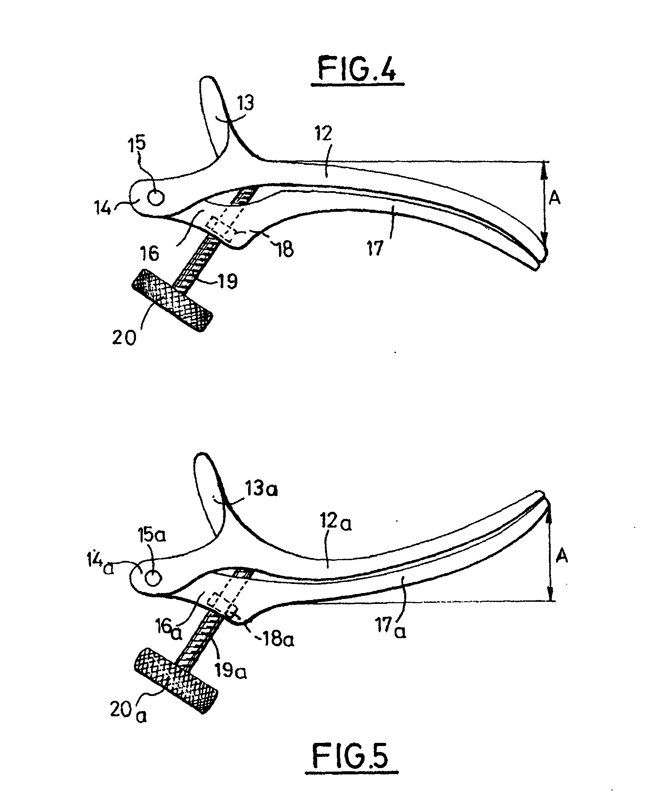

A la figure 4, on a représenté un spéculum physiologique d'un type connu sous l'appellation spéculum COLLIN, le spéculum comprend une première branche 12 à section arquée avec une face convexe extérieure et une face concave intérieure, ladite branche 12 étant terminée par une collerette 13 avec deux ailes latérales 14 sur lesquelles s'articulent les ailes 16 d'une seconde branche 17.

La seconde branche 17 a une section arquée avec une face concave tournée vers la branche 12 et une face convexe extérieure.

Entre les ailes 16 est fixé un écrou 18 dans lequel se visse une vis 19 avec une tête moletée 20.

La vis 19 permet de maintenir les branches écartées et de régler ledit écartement.

Conformément à l'invention, les branches 12 et 17 sont courbées, le déport A, à l'extérieur libre desdites branches, étant de l'ordre de 2 cm.

La figure 5 montre en élévation une variante de réalisation du spéculum COLLIN. Sur cette figure, on a reporté les références utilisées à la figure précédente pour désigner les parties correspondantes, mais affectées de la lettre "a".

Comme on le voit sur cette figure, les branches 12a et 17a sont courbes mais la courbure est orientée dans le sens opposé de celle de la figure 4. Le déport A est sensiblement le même que dans les figures précédentes, soit environ 2 cm.

Bien entendu, l'invention n'est pas limitée aux modes de réalisation qui viennent d'être décrits et représentés. On pourra y apporter de nombreuses modifications de détail sans sortir pour cela du cadre de l'invention, par exemple, les moyens d'écartement des branches et de blocage de celles-ci dans la position angulaire désirée pourraient être différents, tels qu'à crémaillère ou autre.

## Revendications

1. Spéculum pour visualiser le col de l'utérus du type comprenant des première et deuxième branches ou valves (1, 2, 12, 17) articulées l'une sur l'autre par une extrémité suivant un axe (3, 15), l'intérieur étant défini par l'espace entre les branches, chaque branche ayant une section arquée de manière que les faces en regard l'une de l'autre soient concaves, tandis que les faces extérieures sont convexes, des moyens (6, 7, 10, 18, 19, 20) étant insérés entre les branches afin, d'une part, de commander leur écartement et, d'autre part, de les maintenir écartées dans sa position angulaire désirée, et, vu en élévation lorsque l'on regarde dans la direction de l'axe, la première branche étant délimitée par une première ligne extérieure et une première ligne intérieure se rejoignant en une première extrémité libre, la deuxième branche étant délimitée par une deuxième ligne extérieure et une deuxième ligne intérieure se rejoignant en une seconde extrémité libre, la première ligne extérieure étant incurvée en ayant sa concavité tournée vers l'intérieur, **caractérisé en ce que** la deuxième ligne extérieure est incurvée en ayant sa concavité tournée vers l'extérieur.

2. Spéculum suivant la revendication 1, **caractérisé en ce que** la première ligne intérieure a sa concavité tournée vers l'intérieur et la deuxième ligne intérieure a sa concavité tournée vers l'extérieur.

3. Spéculum suivant la revendication 2, **caractérisé en ce que** la concavité de la première ligne intérieure est sensiblement égale à la convexité de la deuxième ligne intérieure

4. Spéculum suivant la revendication 1, 2 ou 3, **caractérisé en ce que** les branches présentent, à leur extrémité libre, un déport (A) de l'ordre de 2 cm par rapport à l'autre extrémité.

5. Spéculum suivant l'une des revendications 1 à 4, **caractérisé en ce que** les branches sont en matière plastique.

## Claims

1. Speculum for displaying the cervix of the uterus of the type comprising first and second branches or valves (1, 2, 12, 17) linked to one another by an end along a pin (3, 15), the interior being defined by the space between the branches, each branch having an arched section so the faces facing one another are concave whereas the outer faces are convex, means (6, 7, 10, 18, 19, 20) being inserted between the branches so as, on the one hand, to control the spacing therebetween and, on the other hand, to set them apart in its desired angular position, and, seen in elevation when viewed in the direction of the pin, the first branch being delimited by a first outer line and a first inner line joined together at a first free end, the second branch being delimited by a second outer line and a second inner line joined together at a second free end, the first outer line being curved as a result of its concavity being turned toward the interior, **characterised in that** the second outer line is curved as a result of having its concavity turned toward the exterior.

2. Speculum according to claim 1, **characterised in that** the first inner line has its concavity turned toward the interior and the second inner line has its concavity turned toward the exterior.

3. Speculum according to claim 2, **characterised in that** the concavity of the first inner line is substantially equal to the convexity of the second inner line.

4. Speculum according to claim 1, 2 or 3, **characterised in that** the branches have, at their free end, an offset (A) of about 2 cm in relation to the other end.

5. Speculum according to any one of claims 1 to 4, **characterised in** the branches are made of plastics material.

## Patentansprüche

1. Spekulum für die visuelle Untersuchung des Uterushalses des Typs, der erste und zweite Arme oder Klappen (1, 2, 12, 17) umfasst, die miteinander über ein Ende entlang einer Achse (3, 15) gelenkig verbunden sind, wobei das Innere von dem Raum zwischen den Armen definiert wird und jeder Arm einen derart gebogenen Querschnitt aufweist, dass die zueinander zeigenden Flächen konkav sind, während die Außenflächen konvex sind, wobei zwischen die Arme Mittel (6, 7, 10, 18, 19, 20) eingesetzt sind, um einerseits ihre Spreizung zu regulieren und sie andererseits in der gewünschten Winkelposition gespreizt zu halten, und wobei, in der Vorderansicht in Richtung der Achse gesehen, der erste Arm von einer ersten äußeren Linie und einer ersten inneren Linie begrenzt wird, die in einem ersten freien Ende zusammentreffen, und der zweite Arm von einer zweiten äußeren Linie und einer zweiten inneren Linie begrenzt wird, die in einem zweiten freien Ende zusammentreffen, wobei die erste äußere Linie mit nach innen gerichteter Konkavität gebogen ist, **dadurch gekennzeichnet, dass** die zweite äußere Linie mit nach außen gerichteter Konkavität gebogen ist.

2. Spekulum nach Anspruch 1, **dadurch gekennzeichnet, dass** die Konkavität der ersten inneren Linie nach innen gerichtet ist und die Konkavität der zweiten inneren Linie nach außen gerichtet ist.

3. Spekulum nach Anspruch 2, **dadurch gekennzeichnet, dass** die Konkavität der ersten inneren Linie im Wesentlichen gleich der Konvexität der zweiten inneren Linie ist.

4. Spekulum nach Anspruch 1, 2 oder 3, **dadurch gekennzeichnet, dass** die Arme an ihrem freien Ende einen versatz (A) in der Größenordnung von 2 cm in Bezug auf das andere Ende aufweisen.

5. Spekulum nach einem der Ansprüche 1 bis 4, **dadurch gekennzeichnet, dass** die Arme aus Kunststoff sind.
